# EUROPEAN PATENT APPLICATION

(11) **EP 3 895 653 A1**
(43) Date of publication of application: **20.10.2021**
(21) Application number: 20382312.5
(22) Date of filing: 17.04.2020
(51) Int. Cl.: A61B 90/40, A61B 90/00, A61B 46/00, A61B 46/23, A61B 46/10, A61B 46/20, A61B 90/20, A61B 90/25, B25J 21/02, A61G 10/00, A61G 15/10

(54) **DEVICE FOR AEROSOL CONTAINMENT IN A DENTAL OFFICE**

(71) Applicant: Pérez Yanini, Juan Carlos, 28223 Pozuelo de Alarón - Madrid (ES); Llansana Fito, Ferran, 07013 Palma de Mallorca (ES); Soler Brullet, Carlos, 08950 Esplugues de Llobregat - Barcelona (ES)
(72) Inventor: Pérez Yanini, Juan Carlos, 28223 Pozuelo de Alarón - Madrid (ES); Llansana Fito, Ferran, 07013 Palma de Mallorca (ES); Soler Brullet, Carlos, 08950 Esplugues de Llobregat - Barcelona (ES)
(74) Representative: Curell Suñol S.L.P.

(57) **Abstract**

Device (1) for aerosol containment in a dental office. Said dental office having a dental chair (2) for accommodating a patient and a microscopy device (3) configured for being placed over said patient's oral cavity. Said device (1) comprising:
- a holding part (100), removably attachable to said microscopy device (3); and
- a shelter part (200), removably attachable to said holding part (100), hanging thereof, thereby forming an aerosol containment shelter (201) over said patient.

Wherein said holding part (100) is configured for allowing the vision through said microscopy device (3) when is attached thereto. Said shelter part (200) comprising three access ports (202), each of said access ports (202) configured for providing an airtight closure at least when an arm is inserted therein.

## Description

### Field of the invention

The invention lies in the field of protection and isolation for dental offices. In particular, for minimizing the possibility of biological contamination between patients or patients and dental professionals, in the case that one patient has a viral or bacteriological infection, especially a respiratory infection or one that could be transmitted due to the aerosols or droplets that are expelled during a dental treatment.

In particular, the invention relates to a device for aerosol containment in a dental office, said dental office having a dental chair for accommodating a patient and a microscopy device configured for being placed over said patient's oral cavity.

### Prior art

Recently, and in particular due to the expansion of infectious diseases worldwide, as in the case of the COVID-19 caused by the SARS-CoV 2 virus, there has been an increment in the security awareness in all aspects of everyday life. Security measures have been deployed in order to minimize the possibility of contagion of such diseases.

In the case of dental offices, the protocols and measures often copy those of other medical areas. For example, using gloves, face masks, suits or other types of personal protective equipment, PPE. Said PPE is used in combination with hygienical protocols including washing the hands and the dental equipment.

Nevertheless, due to the nature of dental treatments, there is an important creation of aerosols coming from the mouth of the patient, in particular, highly incremented by the use of dental equipment, for example dental drills. This equipment acts like spreading means. If the patient has an infection that can be transmitted by said aerosols, the room where the treatment is being performed gets contaminated. After the patient leaves the treatment room, said room cannot be used for some time: firstly, it is needed a time for the aerosols to fall down and settle on the surfaces. According to the available data at the time of writing this document, contaminated aerosols can be present in the air for 30 minutes to two hours. In the case of the SARS-CoV 2, the virus can survive up to 3 hours in aerosol-saturated environments such are the dental treatment rooms, and can contaminate a radius of up to 3-4.5 meters when the droplets fall down. Secondly, the room has to be thoroughly cleaned for decontamination. For example, in the case of SARS-CoV 2, the mean life of the virus on steel surfaces is at least 5.6 hours, and on plastic surfaces 6.8 hours. These two steps should be performed before receiving another patient, otherwise there is a risk that the other patient gets infected. A highly trained cleaning staff is necessary. Even in this case, due to the fact that a treatment room often contains several objects, tools, equipment, furniture, etc. there is a non-zero risk of contagion. Moreover, PPEs or any exposed clothing are also contaminated, and it is often needed to replace said PPEs on a patient-by-patient basis, thus incrementing the need of PPEs. Besides, the massive use of chemical products for cleaning has an environmental impact.

The dental professionals are also exposed to said contamination, even if using PPEs, there is a constant risk of exposure, for example, if the PPEs fail or are not adequately handled.

In addition, there is an important decrement of the availability of the treatment room due to decontamination stages between patients.

Some known solutions have been put in place in order to minimize the aforementioned problems. Two main kind of solutions are known. The first one is based in a transparent screen located over the patient face and mouth. It allows the dental professionals to work, viewing the patient and being able to access to him/her. This kind of barrier decreases the aerosols, but only a part of them. Besides, the screen can be easily be splattered during the treatment. A second kind are vacuum suction systems, having an end over the patient to suction the expelled aerosols. In order to improve the suction some of these solutions use a face mask. The suction not always grants the suction of all the aerosols, and the visibility and access are greatly hindered for the dental professionals. In both cases, a simple error like hitting the suction pump or the screen can lead to a sudden increase of aerosols that are not blocked and will stay in the treatment room.

Since these solutions do not offer a total protection for the dental professionals, in order to minimize the possibility of contagion it is often required that they use a full range of PPE elements: face mask, googles, protective screens, gloves, isolation suits, etc. Even in this case, if the dental treatment takes a long time, the risk of any error that could lead to a contagion for the dental professional increases. Moreover, the comfortability of the dental professionals is also greatly affected.

The aforementioned solutions are particularly problematic for the dental professionals working with vision magnification equipment, in particular microscopy devices, for example, a dental microscope. The first reason is that, in these cases, the treatment typically takes a long period of time, often between 40 minutes and two or even three hours. The second reason is that the known solutions described above do not allow positioning the magnification equipment over the patient's mouth, either because the device itself interferes and blocks the vision through the magnification equipment or because the device needs to be positioned in the same location than the magnification equipment should be. Moreover, if the dental professional is using PPEs such are facial screens, it is not possible to use the binocular tubes of a dental microscope. Therefore, is not possible using the known solutions for dental treatments that require the use of microscopy devices.

Therefore, it is needed an invention for minimizing the possibility of contamination in dental offices and that avoids the aforementioned problems.

### Summary of the invention

The invention is aimed to provide a device for aerosol containment in a dental office of the type stated at the beginning, being able to avoid the problems that have been identified above.

This purpose is achieved by a device for aerosol containment in a dental office of the type indicated at the beginning, characterized in that said device comprises:
- a holding part, removably attachable to said microscopy device; and
- a shelter part, removably attachable to said holding part, hanging thereof, thereby forming an aerosol containment shelter over said patient;
wherein said holding part is configured for allowing the vision through said microscopy device when is attached thereto; said shelter part comprising three access ports, each of said access ports configured for providing an airtight closure at least when an arm is inserted therein.

Therefore, the device surrounds the patient and, at the same time, makes possible the dental treatment even in the case where microscopy devices have to be used. Indeed, the dedicated holding part has the technical effect of providing a viewport from the microscopy device towards the patient because it is configured in such a way that does not interfere with the vision through the microscopy device. A skilled person will have no problems in finding different configurations allowing said characteristic, by the way of non-limiting examples, the holding part being a hollow cylinder or a solid transparent element attachable to the lens of the microscopy device. Since the dental professionals can access directly to the patient through said access ports without further impediments, the treatment possibilities are not hindered. In particular, two of the access ports are aimed for the dental operator and are preferably located one at each side of the patient, so that the dental operator can insert his/her arms from behind the head of the patient. Another access port is aimed for the dental auxiliary, so that he/she can manipulate a suction pump that is used during dental treatment.

In most of the infection diseases, for example, in the case of COVID-19, the highest risk of contamination is due to the aerosols expelled during the dental treatment. The device facilitates the containment of said aerosols or droplets in a volume of space around the patient mainly defined by the shelter part. This containment is further improved by the airtight closure of the access ports. Therefore, the contamination is mostly restricted to said containment shelter. The latter also affects the PPEs of the dental professionals since only the gloves could be eventually exposed inside the containment shelter.

Moreover, since the possible contamination affects basically to the surfaces of the elements that are inside the containment shelter, among others, the patient, any tool, the dental chair, the floor and the internal surface of the shelter part. Therefore, only those surfaces need to be thoroughly cleaned after the dental treatment has finished. In addition, the aerosols are contained inside the shelter and not in the whole room. This greatly simplifies the decontamination stages between patients and minimizes the risk of contamination. In some embodiments the shelter part is disposable, further decreasing the time needed for decontamination because it only has to be replaced for the next patient. In other embodiments, the shelter part can be sterilized using a dedicated procedure, for example, using an autoclave. The latter option also makes possible to decrease the time needed for decontamination stages while producing less waste material. Indeed, once one dental treatment has been finished for one patient, the shelter part used can be sterilized while using another shelter part for a new patient.

Once installed, the shelter part hangs from the holding part, thereby simplifying the installation of the device. Indeed, no special equipment is needed in order to deploy the device for a patient: the patient can simply sit in the dental chair, the shelter part is then placed over the patient and connected to the holding part which in general will be already attached to the microscopy device. A further advantage is that the device can be used in a wide range of dental offices since no special arrangements are necessary.

In one preferred embodiment, said airtight closure of said access ports is, each independently, provided by one of the list consisting of:
- an airtight arm glove formed towards the interior of said aerosol containment shelter;
- an elastic sleeve, preferably made of silicone or latex; and
- a plastic sleeve having an elastic rubber ring.

Therefore, each access port can have a different type of means for granting the airtight closure if considered necessary. The arm gloves formed towards the interior of the shelter have the advantage that they provide an airtight closure even if the arm is not inserted thereto, further allowing to remove and re-insert the arm without affecting the airtight closure. In addition, they provide a high level of protection because there is a physical barrier between the dental professional using the glove and the interior of the containment shelter, thus maximizing the safety of the device. Nevertheless, this kind of ports have the drawback that it is not possible to introduce any item inside the containment shelter though the access port. This operation can be useful in the case that, for example, any tool to be used is initially arranged outside the shelter. A further drawback is a greater manufacturing cost for the shelter part. By contrast, a plastic sleeve having an elastic rubber ring that gently squeezes the arm inserted thereto has a low fabrication cost and is particularly suitable for disposable shelter parts. It also has the advantage that items can be introduced through the access port. The level of air tightness in this case is lower than in the case of the arm glove. Elastic sleeves, such those made of latex or silicone, provide a level of air tightness which is generally higher than the previous preferred option and they also allow introducing items through the port. In a preferred embodiment, at least one of the access ports, in particular one aimed for the dental operator, is provided with an arm glove as described above. This configuration allows the dental operator to remove the arm, for example, for manipulating the position of the microscopy device or its focus. The arm can be re-inserted again in the access port without compromising the containment. Preferably, two of the access ports, in particular the ones aimed for the dental operator, are provided with corresponding arm gloves, thus improving the possibilities of actions for the dental operator, and irrespective if he is right-handed or left-handed.

Preferably, said holding part is rigid and has hollow tubular form, having a top end provided with a microscope adapter configured to be removably attachable to said microscopy device, and a bottom end provided with a shelter adapter; wherein said shelter part has a holding adapter, configured to be removably attachable to said shelter adapter. Therefore, the resulting element does allow the vision through the microscopy device. At the same time, the structure is robust and has a simplified configuration, thus, the manufacturing costs are minimized. In addition, the microscope adapter can be selected in order to adapt to different types of microscopy devices. The same applies for the shelter adapter. Preferably, said holding part is configured so that to avoid internal reflections of any light coming from the microscopy device. In general, the microscopy devices used in dental treatment are provided with a lamp that emits high-intensity light towards the direction of vision, for example, using high power LEDs. If the light is reflected towards the lens, it can create halos or internal reflections that decrease the quality of the image. Solutions for avoiding reflections are known in the field of optics, for example, using non-reflective coatings or materials, or using adequate internal geometries the holding part for avoiding such reflections.

Preferably, said microscope adapter is configured to fit around a focus of said microscopy device. Said focus ring, or in some cases, a zoom ring, is often located at the bottom end of the microscopy device. Fitting the holding part to that bottom end has the advantage that the holding part itself can be used for protecting the lens of the microscopy device. In addition, the ring can be operated by rotating the holding device.

Preferably, said microscope adapter further comprises microscope fastening means, configured to fasten said microscope adapter to said microscopy device, thus improving the attachment strength between the microscopy device and the holding part that, in turn, supports the shelter part. Said microscope fastening means preferably comprise at least one fastening screw, thus facilitating the fastening and adapting to different models or variations in the shape of the microscopy device. Said fastening screw has preferably a flat or dull end, so that the microscopy device is not damaged.

Preferably, said holding part comprises an upper section, jointly rotatable with said focus ring, and a lower section, wherein rotation isolation means are provided between said upper section and said lower section, said rotation isolation means configured to isolate the lower section from the rotation of the upper section. The upper part can be attached to the focus ring of the microscopy device. Any rotation of the focus ring results in a rotation of the upper section of the holding device. Thanks to the isolation means, the rotation of the upper section is not transferred to the lower section. Therefore, when the shelter part is attached to the bottom end of the holding part, the rotation in the focus ring does not affect to the shelter part. Preferably comprising a rotation bearing, in particular, provided in the contour of the holding part, thus providing a smooth operation in case of rotation.

Preferably, said holding part further comprises a suction port, configured for removably attaching a surgical suction pump. The suction pump removes the aerosols and droplets expelled from the mouth of the patient, making it more difficult for said droplets to reach the lens or to cause splattering that could hinder the vision through the microscopy device. Preferably, said suction port is provided in the lower section of the holding part, so that any rotation of the upper part is not transferred to the suction port. Preferably, said suction port is provided with closing means, so that the suction port can be airtightly closed in the case that no suction pump is used, for example, if the suction pump is already provided near the mouth.

Preferably, said holding part further comprises a slot for receiving a transparent isolation plate, said slot and said isolation plate configured for jointly block the passage of aerosols or droplets when said isolation plate is inserted into said slot. Therefore, the internal surfaces in the holding part that are exposed to contamination are only those located under the isolation plate. Said isolation plate further protects the lens of the microscopy device. Moreover, in the case that it gets splattered hindering the vision, it is possible to remove the isolation plate, clean it, and insert it again. The isolation plate is preferably configured so that it can be sterilized using a standard sterilization method such autoclave. In a preferred embodiment the isolation plate is made of glass, which is sterilizable and does not adversely affects the vision through it.

Preferably, the zone of the shelter part that covers the patient face is transparent, so that it makes it possible for the dental professionals to see the patient during the treatment. In an alternative embodiment, said shelter part is transparent. This improves the comfortability for the patient, minimizing claustrophobic reactions, and also makes it possible for the dental professionals to see the patient during the treatment.

Preferably, said shelter part comprises a further port, said further port provided with closing means configured for providing an airtight closure to said further port, said closing means openable to allow the passage of an arm; said further port configured for providing an airtight interface when an arm is inserted therein. Therefore, it is possible to use the further port in the case that is necessary to introduce an object inside the containment shelter, for example, a tool that becomes needed during the dental treatment. In normal conditions the further port is closed, blocking the passage from inside the containment shelter to the outside. After an arm is introduced thereto, it provides an airtight closure. Preferred means for providing said airtight closure in ports have been discussed above, by means of non-limiting examples, latex, silicone sleeves, or plastic sleeves provided with a rubber ring.

Preferably, said shelter part is formed by a flexible sheet made of polymeric material suitable for blocking the passage of aerosols. Therefore, the shelter part is easy to transport, store and deploy, and has a low fabrication cost.

Preferably, said device further comprises at least two supporting elements, configured for supporting said flexible sheet away from the body of the patient, thus giving more space to the patient, thereby improving comfortability. In addition, it also minimizes the possibility that a movement of the patient affects the stability of the structure of the shelter part, for example, if the patient suddenly kicks that movement is not transferred to the shelter part and it is not possible that it detaches from the holding part, falling over the patient. In a preferred embodiment, it comprises four supporting elements, thus creating a shelter structure with four supporting points, in particular away from the feet and the shoulders of the patient. In an alternative embodiment, said device comprises two supporting elements, provided outside of said containment shelter at both sides of the position of the patient's hips or feet. The latter embodiment keeps the flexible sheet away from the patient but does not difficult the access to the dental professionals.

Preferably, each of said supporting elements has an attachment element configured for attaching and holding a corresponding attachment element provided in the outer side of said shelter part. The support of the shelter part is done from the outside, therefore, the supporting elements themselves are not inside the containment shelter and do not get contaminated. Preferably, each of said supporting elements is a pillar. Pillars can be made at fixed height or can be extendable to adapt to different working conditions. Preferably, they are movable pillar, so that they can be used in the treatment room only when needed and can be located according to the working environment and the body dimensions of the patient.

Preferably, said device further comprises a shelter floor, attachable to said shelter part, thereby creating said aerosol confinement shelter in the volume formed between said shelter part and said shelter floor. Therefore, the floor of the treatment room does not get contaminated, thereby simplifying the decontamination stage.

Preferably, said shelter floor is formed by a flexible sheet made of polymeric material suitable for blocking the passage of aerosols, having the aforementioned advantages. Preferably, it is transparent as in the case of the shelter sheet. Preferably, said shelter floor has a shape allowing to cover said dental chair. The latter is particularly advantageous for avoiding contamination of the dental chair, which is a complex structure and can be difficult or time consuming to clean it thoroughly.

### Brief description of the figures

Further advantages and features of the invention will become apparent from the following description, in which, without any limiting character, preferred embodiments of the invention are disclosed, in reference to the accompanying figures:
Fig. 1 shows a lateral view of a first embodiment of a device according to the invention. According to common drawing conventions, relevant hidden parts have been represented with dashed lines.
Fig. 2 is a lateral section view of the holding part of the device of Fig. 1.
Fig. 3A and 3B are section top views of the holding part in the zone of the transparent plate. Fig 3A shows the plate outside of the slot in holding part, and Fig. 3B shows the plate inserted in said slot.
Fig. 4 is a section view showing a type of sleeve for the access ports.
Fig. 5 is a section view showing another type of sleeve for the access ports.
Fig. 6 is a lateral view showing another embodiment of the device according to the invention.
Fig. 7 is a section view showing a configuration for the further access port depicted in Fig. 6.
Fig. 8 to Fig. 11 are lateral views showing different embodiments of the device according to the invention.

### Detailed description of embodiments o the invention

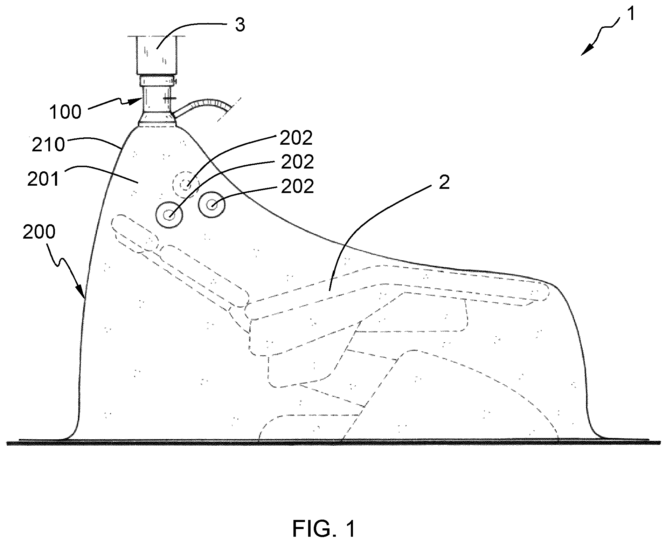

Fig. 1 shows a first exemplary embodiment of a device 1 for aerosol containment in a dental office according to the invention. The dental office has a treatment room having a dental chair 2 for accommodating a patient and a microscopy device 3, in the case of the embodiments shown here, a dental microscope 3 that is configured for being placed over said patient's oral cavity. Given the form of the dental chair 2 and the location of the dental microscopy 3, the position of the patient on said chair will be evident. In particular, in the figures, the patient's head will be located in the left side of the drawing, under the dental microscopy 3. Therefore, and for the sake of clarity, the patient has not been represented in the figures.

The device 1 shown in Fig. 1 has a holding part 100, removably attachable to the dental microscope 3. Said holding part 100 is configured for allowing the vision through the microscope 3 when is attached thereto. The device 1 also has a shelter part 200, removably attachable to said holding part 100. When it is attached, it hangs thereof, thereby forming an aerosol containment shelter 201 over said patient when he or she is sitting on the dental chair 2. In the embodiments shown in the figures, the shelter part 200 is transparent and formed by a flexible sheet 210 made of polymeric material suitable for blocking the passage of aerosols. In addition, the shelter part 200 in the embodiments is disposable and aimed for a single use. Nevertheless, other embodiments where the shelter part 200 is reusable after a decontamination process can also be envisaged.

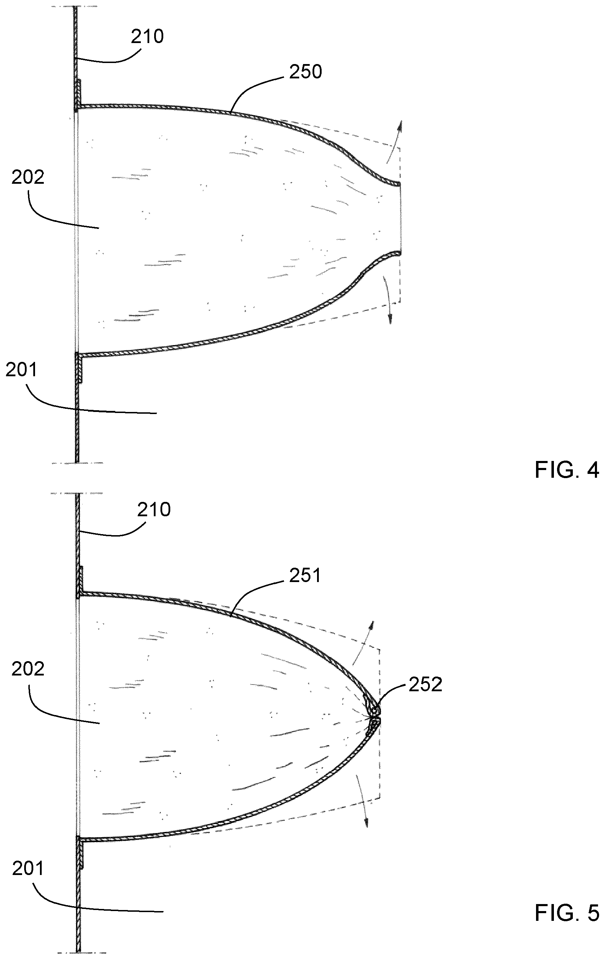

Fig. 1 shows that the shelter part 200 has three access ports 202. Each of said access ports 202 is configured for providing an airtight closure when an arm is inserted therein. Two of said access ports 202 are symmetrically located at both sides of the shelter part 200 and are aimed for the dental operator. According to this description, one of said two access ports 202 should be hidden in the figure. Nevertheless, for the sake of clarity, the hidden access port 202 has been drawn with an offset so it can be shown in the figure. The same consideration has been made for the equivalent figures of the other embodiments of the invention. The third access port 202 is aimed for the dental auxiliary. For all three access ports 202, the airtight closure is provided by a silicone elastic sleeve 250. Fig. 4 shows an example of said type of sleeve. Other possibilities can be also envisaged, for example, latex sleeves or sleeves made of equivalent elastic materials.

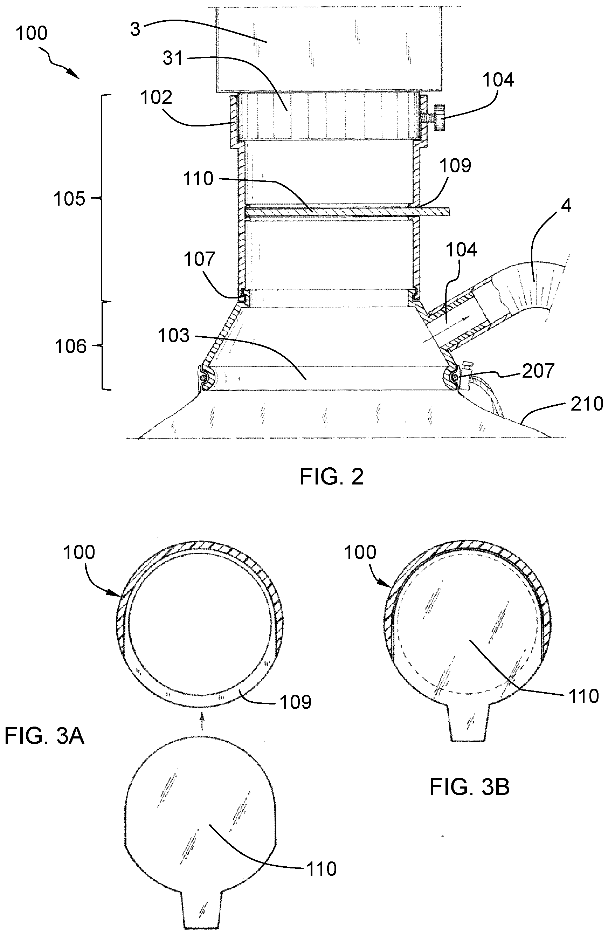

Fig. 2 shows a detailed view of the holding part 100. Said holding part 100 is hollow and has a tubular form, thus allowing the vision through the dental microscope 3 to the patient's mouth when the holding part 100 is attached to the microscope 3. The skilled person will have not problems in selecting the internal diameter of the hollow tube in order to avoid vignetting, for example, by simple experimentation with different sizes or by using the common general knowledge in the field of optics for calculating the correct size taking into consideration factors such are the characteristics of the microscope 3 and the length of the tubular part 100.

The top end of the holding part 100 is provided with a microscope adapter 102 configured to be removably attachable to the dental microscope 3. In particular, said microscope adapter 102 is configured to fit around a focus ring 31 of the dental microscope 3, as shown in Fig. 2. In addition, the microscope adapter 102 further comprises fastening means 104 in the form of a fastening screw that allows fastening the microscope adapter 102 to the microscopy device 3, and securely fix it thereto.

The bottom end of the holding part 100 is provided with a shelter adapter 103. The shelter part 200 has a holding adapter 207, configured to be removably attachable to said shelter adapter 103. In the case of this first embodiment, the shapes of both adapters 103 and 207 are complementary, so that the shelter adapter 103 can be snap-fitted into the holder adapter. A fastening strap or equivalent means can be used for tightly secure one against the other. The configuration represented in Fig. 2 is a possible example, but different configurations can also be envisaged for a secure attachment between the shelter adapter 103 and the holding adapter 207, by way of non-limiting examples, a screwed connection or a bayonet connection.

As shown in Fig. 2, the holding part 100 is divided in two sections: an upper section 105, and a lower section 106. The upper section 105 is provided with the microscope adapter 102 in its top end which can be attached to the microscope's focus ring 31, thus being jointly rotatable with said focus ring 31. The upper section 105 is connected to the lower section 106 by means of rotation isolation means 107, configured to isolate the lower section 106 from the rotation of the upper section 105. The simplified exemplary isolation means 107 depicted in Fig. 2 is formed by two circumferential grooves, one in each section 105, 106, and two corresponding cantilevered ends of each of the sections 105, 106. For each section, its cantilevered end is inserted into the corresponding groove of the other section and can slide therein, thus allowing rotation of one section with respect to the other section. Other possible isolation means 107 can be envisaged, by the way of a non-limiting example, using rotation bearings in order to provide a smooth rotation.

The holding part 100 further comprises a suction port 108, located in the lower section 106 and configured for removably attaching a surgical suction pump 4.

In addition, the holding part 100 further comprises a slot 109, arranged horizontally in the holding part 100 and configured for receiving a transparent isolation plate 110 made of glass. The slot 109 and the isolation plate 110 are configured so that, when the isolation plate 110 is inserted into the slot 109, they jointly block the passage of aerosols or droplets coming from the containment shelter 201 towards the lens of the dental microscope 3. Fig. 3A and Fig 3B are section top views of the detail of the slot 109 and the isolation plate 10. Fig. 3A shows the isolation plate 110 outside the slot 109 and Fig. 3B shows the same isolation plate 110 once inserted into the slot 109, and in the same position that is shown in Fig. 2.

Other embodiments of the device 1 according to the invention are disclosed hereinafter. These embodiments share most of the features disclosed in the first embodiment above. Therefore, only the differentiating features will be described in detail. For the sake of brevity, common features shared with the first embodiment disclosed above will not be described again hereinbelow.

Fig. 5 shows a detail of a second embodiment of the device 1 of the invention. In the case of this embodiment, the airtight closure of each of the access ports 202 is provided by plastic sleeve 251 having an elastic rubber ring 252.

In another embodiment not shown in the figures, said airtight closure is provided by an airtight arm glove formed towards the interior of said aerosol containment shelter 201. In still other embodiments, not all the access ports 202 have the same configurations, for example, the two access ports 202 aimed for the dental operator are provided with arm gloves, while the access port 202 aimed for the dental auxiliary is provided with a silicone elastic sleeve 250. Different combinations can be also envisaged.

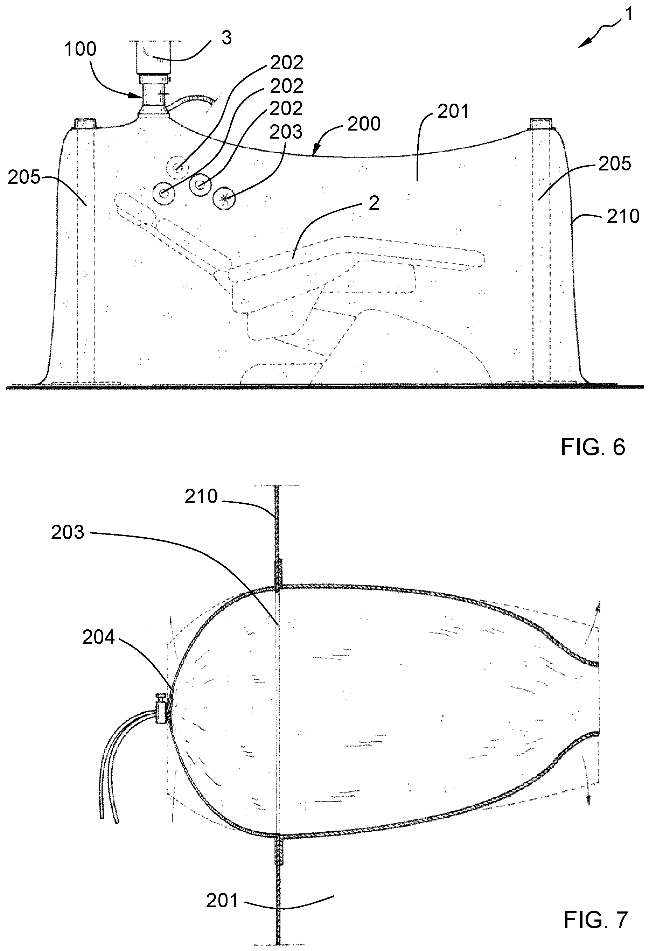

Fig. 6 shows a third embodiment of the device of the invention wherein the shelter part 200 comprises a further port 203 which is shown in more detail in Fig. 7. Said further port 203 is provided with closing means 204 configured for providing an airtight closure to said further port 203, said closing means 204 openable to allow the passage of an arm. In particular, the closing means 204 is a plastic sleeve having an adjustable strip at its end. The adjustable strip can be fastened for closing the further port 203 or loosened for opening the further port 203. The further port 203 is configured for providing an airtight interface when an arm is inserted therein, by means of a silicone sleeve oriented towards the interior of the containment shelter 201. As shown in Fig. 6, the device 1 of the third embodiment further comprises four supporting elements 205, configured for supporting said flexible sheet 210 away from the body of the patient. Each of the supporting elements 205 is a movable pillar provided to support the flexible sheet 210 from the interior of the containment shelter 201, therefore simplifying the deployment of the device 1.

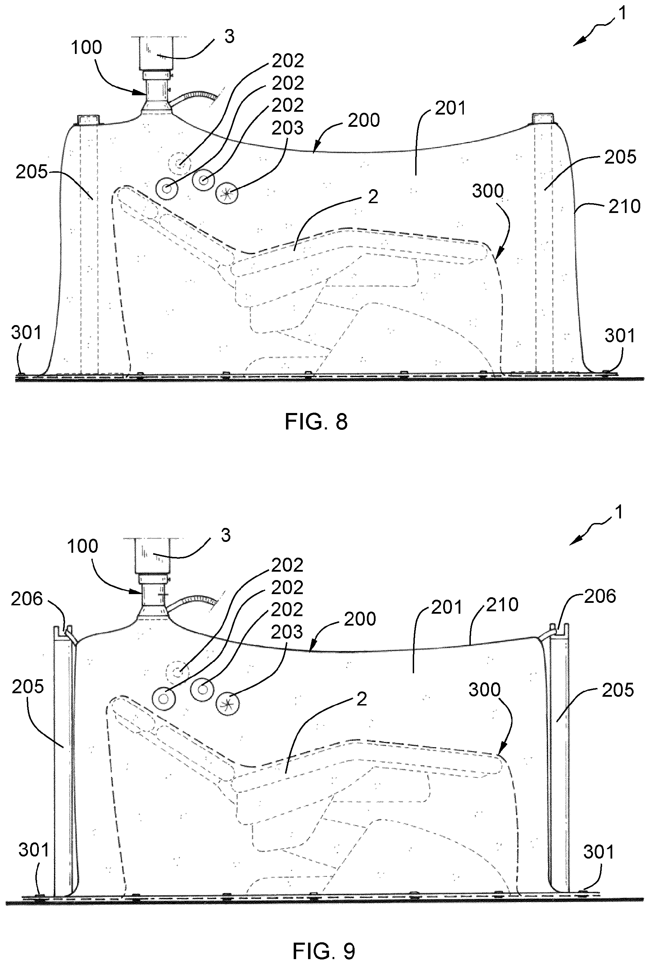

Fig. 8 shows a fourth embodiment of the device 1 of the invention. This embodiment is based on the third embodiment described above, further comprising a shelter floor 300, attachable to said shelter part 200, thereby creating said aerosol confinement shelter 201 in the volume formed between said shelter part 200 and said shelter floor 300. In particular, the shelter floor 300 is formed by a flexible sheet made of the same material that the flexible sheet 210 of the shelter part 200, i.e. a transparent polymeric material suitable for blocking the passage of aerosols. As shown in Fig. 8, the shelter floor can be deployed covering the dental chair 2.

Fig. 9 shows a fifth embodiment of the device of the invention. This embodiment is similar to the fourth embodiment of Fig. 8 but wherein four the movable pillars that are the supporting elements 205 are located outside of the containment shelter 201. Each of the pillars 205 has an attachment element 206 in the form of a groove at the top end of the pillar 205. The shelter part 200 is provided with four corresponding attachment elements in the form of external plastic D rings that can be attached to the attachment elements 206 of the pillars 205.

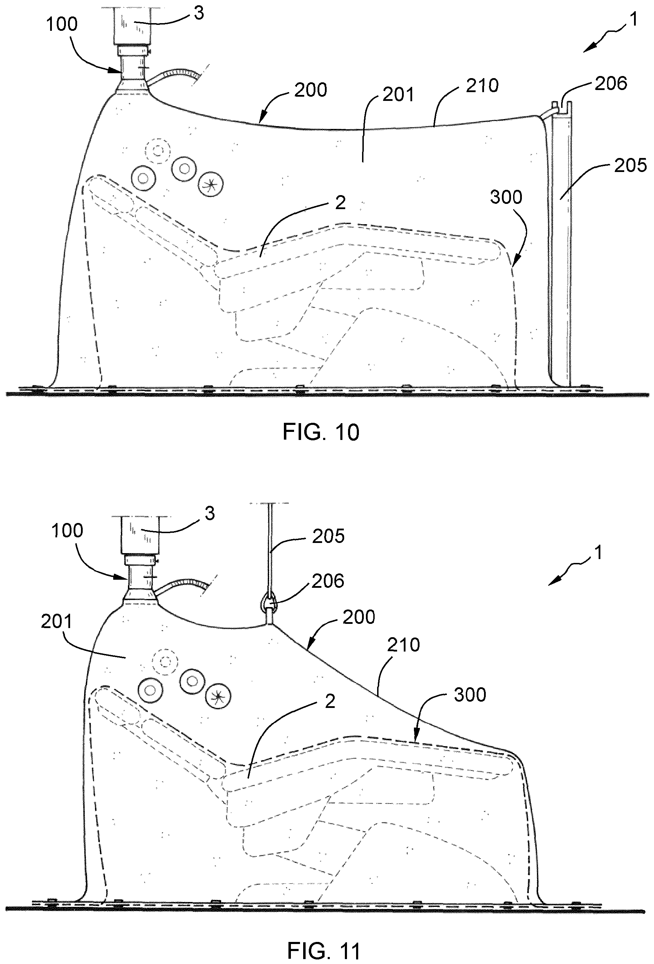

Fig. 10 shows a sixth embodiment of the device of the invention, which is based on the fifth embodiment shown in Fig. 9, wherein the device only has two supporting elements 205 in the form of movable pillars. In this embodiment, the two supporting elements 205 are located in the right part of the figure, corresponding to zone of the feet of the patient.

Fig. 11 shows a seventh embodiment of the device of the invention. This embodiment is based on the sixth embodiment shown in Fig. 10, wherein the two supporting elements 205 instead of pillars are two straps hanging from above the device 1. The attachment element 206 at the end of each strap 205 is a carabiner. Besides, the horizontal location of the supporting elements 205 corresponds to the zone of the hips of the patient.

In other embodiments not shown in the figures, the device further comprises air renewal means in the form of one air renewal port provided in the shelter part 100.

## Claims

1. Device (1) for aerosol containment in a dental office, said dental office having a dental chair (2) for accommodating a patient and a microscopy device (3) configured for being placed over said patient's oral cavity;
**characterized in that** said device (1) comprises:
- a holding part (100), removably attachable to said microscopy device (3); and
- a shelter part (200), removably attachable to said holding part (100), hanging thereof, thereby forming an aerosol containment shelter (201) over said patient;
wherein said holding part (100) is configured for allowing the vision through said microscopy device (3) when is attached thereto;
said shelter part (200) comprising three access ports (202), each of said access ports (202) configured for providing an airtight closure at least when an arm is inserted therein.

2. Device (1) according to claim 1, **characterized in that** said airtight closure of said access ports (202) is, each independently, provided by one of the list consisting of:
- an airtight arm glove formed towards the interior of said aerosol containment shelter (201);
- an elastic sleeve (250), preferably made of silicone or latex; and
- a plastic sleeve (251) having an elastic rubber ring (252);
preferably at least one of said access ports (202) is provided with one of said airtight arm glove; more preferably, two of said three access ports (202) are each provided with one of said airtight arm glove, and the other of said access ports (202) is provided with one of said elastic sleeve (250) or one of said plastic sleeve (251).

3. Device (1) according to any of the claims 1 or 2, **characterized in that** said holding part (100) is rigid and has hollow tubular form, having a top end provided with a microscope adapter (102) configured to be removably attachable to said microscopy device (3), and a bottom end provided with a shelter adapter (103); wherein said shelter part (200) has a holding adapter (207), configured to be removably attachable to said shelter adapter (103).

4. Device (1) according to claim 3, **characterized in that** said microscope adapter (102) is configured to fit around a focus ring (31) of said microscopy device (3), preferably, said microscope adapter (102) further comprising microscope fastening means (104), configured to fasten said microscope adapter (102) to said microscopy device (3), said microscope fastening means (104) preferably comprising at least one fastening screw.

5. Device (1) according to claim 4, **characterized in that** said holding part (100) comprises an upper section (105), jointly rotatable with said focus ring, and a lower section (106), wherein rotation isolation means (107) are provided between said upper section (105) and said lower section (106), said rotation isolation means (107) configured to isolate the lower section (106) from the rotation of the upper section (105), preferably said isolation means (107) comprising a rotation bearing.

6. Device (1) according to any of the claims 3 to 5, **characterized in that** said holding part (100) further comprises a suction port (108), configured for removably attaching a surgical suction pump (4).

7. Device (1) according to any of the claims 3 to 6, **characterized in that** said holding part (100) further comprises a slot (109) for receiving a transparent isolation plate (110), preferably made of glass, said slot (109) and said isolation plate (110) configured for jointly block the passage of aerosols or droplets when said isolation plate (110) is inserted into said slot (109).

8. Device (1) according to any of the claims 1 to 7, **characterized in that** it further comprises air renewal means; configured for renewing the air inside the containment shelter (201), in particular, with air or O₂ enriched air.

9. Device (1) according to any of the claims 1 to 8, **characterized in that** said shelter part (200) is transparent.

10. Device (1) according to any of the claims 1 to 9, **characterized in that** said shelter part (200) comprises a further port (203), said further port (203) provided with closing means (204) configured for providing an airtight closure to said further port (203), said closing means (204) openable to allow the passage of an arm; said further port (203) configured for providing an airtight interface when an arm is inserted therein.

11. Device (1) according to any of the claims 1 to 10, **characterized in that** said shelter part (200) is formed by a flexible sheet (210) made of polymeric material suitable for blocking the passage of aerosols.

12. Device (1) according to claim 11, **characterized in that** it further comprises at least two supporting elements (205), configured for supporting said flexible sheet (210) away from the body of the patient.

13. Device (1) according to claim 12, **characterized in that** each of said supporting elements (205) has an attachment element (206) configured for attaching and holding a corresponding attachment element provided in the outer side of said shelter part (200); preferably, each of said supporting element (205) is a pillar, more preferably a movable pillar.

14. Device (1) according to any of the claims 1 to 13, **characterized in that** it further comprises a shelter floor (300), attachable to said shelter part (200), thereby creating said aerosol confinement shelter (201) in the volume formed between said shelter part (200) and said shelter floor (300).

15. Device (1) according to claim 14, **characterized in that** said shelter floor (300) is formed by a flexible sheet made of polymeric material suitable for blocking the passage of aerosols, preferably transparent, and preferably having a shape allowing to cover said dental chair (2).
